(19) Europäisches Patentamt

European Patent Office

Office européen des brevets

(11) EP 0 795 129 B1

(12) EUROPEAN PATENT SPECIFICATION

(45) Date of publication and mention
of the grant of the patent:
**24.01.2001   Bulletin 2001/04**

(51) Int Cl.[7]: **G01N 33/487**, G01N 15/04,
G01N 21/25, G01N 21/31,
G01N 33/49

(21) Application number: **95938498.3**

(22) Date of filing: **30.11.1995**

(86) International application number:
**PCT/GB95/02786**

(87) International publication number:
**WO 96/17243 (06.06.1996 Gazette 1996/26)**

(54) **APPARATUS FOR ANALYSING BLOOD AND OTHER SAMPLES**

VORRICHTUNG ZUR ANALYSE VON BLUT UND ANDEREN PROBEN

APPAREIL D'ANALYSE DU SANG ET D'AUTRES SUBSTANCES

(84) Designated Contracting States:
**DE FR GB**

(30) Priority: **30.11.1994   GB 9424218**

(43) Date of publication of application:
**17.09.1997   Bulletin 1997/38**

(73) Proprietor: **ZYNOCYTE LIMITED**
**Glasgow G20 0SP (GB)**

(72) Inventors:
  • **CLAMPITT, Roger**
    **Hemel Hempstead, Hertfordshire HP1 3DD (GB)**
  • **HAZELWOOD, Stephen**
    **Whiteinch, Glasgow G14 9QD (GB)**

(74) Representative: **Pacitti, Pierpaolo A.M.E. et al**
**Murgitroyd and Company**
**373 Scotland Street**
**Glasgow G5 8QA (GB)**

(56) References cited:
**EP-A- 0 210 417          EP-A- 0 392 475**
**WO-A-94/18557          DE-A- 3 908 114**

• **BIOMEDIZINISCHE TECHNIK, vol. 30, no. 1/2,
February 1985, BERLIN DE, pages 18-23,
XP002000146 J. BURNFEINDT, ET AL.:
"ANGEWANDTE OPTISCHE
UNTERSUCHUNGEN IM NAHEN INFRAROT-
UND ROTBEREICH AN HUMANVOLLBLUT;
ANWENDUNGSBEISPIELE"**
• **PATENT ABSTRACTS OF JAPAN vol. 11, no. 164
(P-580), 27 May 1987 & JP,A,62 000838
(KAWASUMI LAB INC), 6 January 1987,**

**Description**

This invention relates to an apparatus for analysing blood and other samples by means of automated sample analysis during centrifugation.

In our published International Patent Application WO 94/8557 there is described an apparatus intended principally for the analysis of blood for diagnostic purposes. In that apparatus there is inter alia a rotor disc adapted to receive a number of capillary haematocrit tubes each in a corresponding slit. An arm pivoted to a point clear of the rotor disc carries a light source above the rotor disc and an aligned light sensor below the rotor disc. By combining rotation of the rotor disc with pivotal movement of the arm, the haematocrit tubes can be scanned, and by processing the output signal of the light detector one may derive parameters of interest for each sample, such as the packed cell volume and white cells.

The present invention is concerned, in one aspect, with an improved apparatus of the same general nature.

It is also known in the prior art to measure haemoglobin (Hb) concentration by mixing sample blood with a reagent which undergoes a colour change in dependence on Hb concentration. For accuracy, however, it is then necessary to use a spectrophotometer or colourimeter; this is fairly expensive, and requires a calibration procedure which would be technically demanding in a practice environment.

The present invention, in one aspect, provides an analysis apparatus which includes a centrifuge rotor adapted to receive at least one sample in a transparent holder, the rotor being apertured to allow light to be transmitted through the sample and the rotor, and a scanning arm arranged to traverse the rotor carrying light source means and light detector means on respective sides of the rotor; and in which the light source means comprises a first light source for detection of sample component interfaces and a second light source for colourimetric inspection of at least one sample component.

Preferably, the first light source is an infrared light source and the second light source is a visible light source.

The infrared light source is preferably an infrared laser.

In a particularly preferred form of this aspect of the invention, the sample is of blood, the visible light source is used for examining blood plasma, and the visible light source comprises a plurality of substantially monochromatic light sources, most preferably sources of red, green and blue light at wavelengths of, for example, 625, 567 and 470 nm.

Conveniently, the visible light source may be provided by a single, multi-colour LED with the colours switched sequentially during the scanning process.

The apparatus includes a reference sample container on the rotor containing a reference material (for example, water) of known optical qualities, whereby signals representing light transmission through the sample(s) may be calibrated against signals representing light transmission through the reference.

From another aspect, the present invention provides a haemoglobin photometer comprising means for receiving a transparent cell containing a blood sample mixed with a haemoglobin reagent, illumination means for directing substantially monochromatic light at the sample cell, and detector means arranged to receive light passing through the sample cell to generate a signal representing the optical density of the cell contents.

Preferably, the illumination means comprises a LED operating at a peak wavelength of, preferably, about 560 nm.

The illumination means preferably further comprises collimation means causing the light directed at the sample cell to be substantially parallel.

The detector means may suitably comprise a converging lens directing the transmitted light onto a photodiode.

The illumination means and detector means preferably include optical stops dimensioned and positioned such that light emitted from the whole emitting area of the LED is received within the active surface of the photodiode.

In a particularly preferred feature of this aspect of the invention, the photometer includes calibration means operating to calibrate a detected optical density to a predetermined standard.

Preferably, the calibration means operates by comparing the output of the detector means with and without a sample cell in position.

From another aspect, the invention provides a blood analyzer comprising a centrifuge for separating a blood sample by centrifugation into packed red cells, packed white cells, and plasma; first optical means for measuring the proportions of red cells, white cells and plasma in the sample; photometer means for measuring haemoglobin concentration in a sample of the same blood; and computer means for deriving a number of parameters of interest from said measurements.

Said parameters may suitably be:

Total packed cell volume (PCV)
White blood cell volume (WBC)
Haemoglobin concentration (Hb), and
Mean corpuscular haemoglobin concentration (MCHC) where

$$MCHC = \frac{Hb\ (g/dl)}{PCV\ (\%)} \times 100\ g/dl$$

The analyzer further comprises second optical means for inspecting the plasma component to measure one or more parameters of interest therein.

The second optical means is arranged to measure optical density at each of a plurality of visible wavelengths, suitably wavelengths of approximately 625, 570 and 470 nm.

The parameters of interest are preferably icterus, lipaemia and haemolysis.

An embodiment of the invention will now be described, by way of example only, with reference to the drawings, which:-

Fig. 1 is a schematic block diagram of an apparatus embodying the invention for use in analysing blood;

Fig. 2 is a schematic plan view of a centrifuge rotor and scanning head forming part of the apparatus;

Fig. 3 is a schematic side view of the mechanism of Fig. 1;

Fig. 4 is a partial section to an enlarged scale showing one part of Figs. 2 and 3 in greater detail;

Fig. 5 illustrates a centrifuged blood sample;

Figs. 6A and 6B are curves illustrating output signals;

Fig. 7 is a cross-sectional view of a haemoglobin photometer forming part of the apparatus; and

Fig. 8 illustrates features of the geometry of the parts shown in Fig. 7.

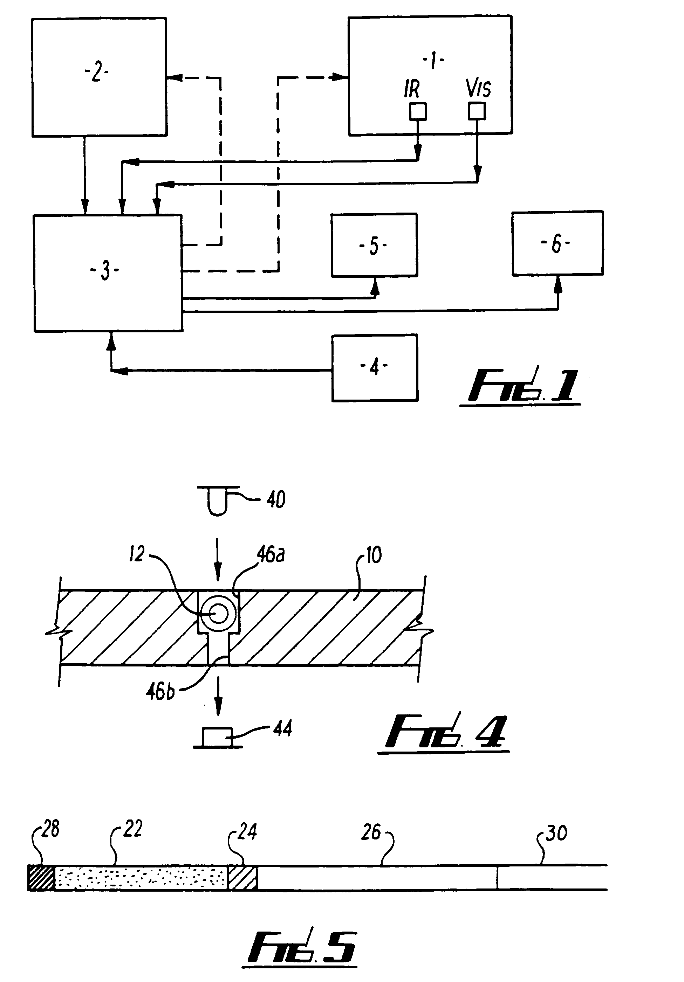

Referring to Fig. 1, the preferred form of the invention is a blood analyser comprising a centrifuge 1 and a photometer 2. The apparatus is controlled by a microprocessor 3 with user input via a keyboard 4 and data output by display 5 and/or printer 6.

The photometer 2 provides an output signal to the microprocessor 3 which is representative of haemoglobin concentration.

The centrifuge 1 includes two optical arrangements, as will be described, providing respective output signals to the microprocessor 3 from which signals a number of blood-related parameters can be derived.

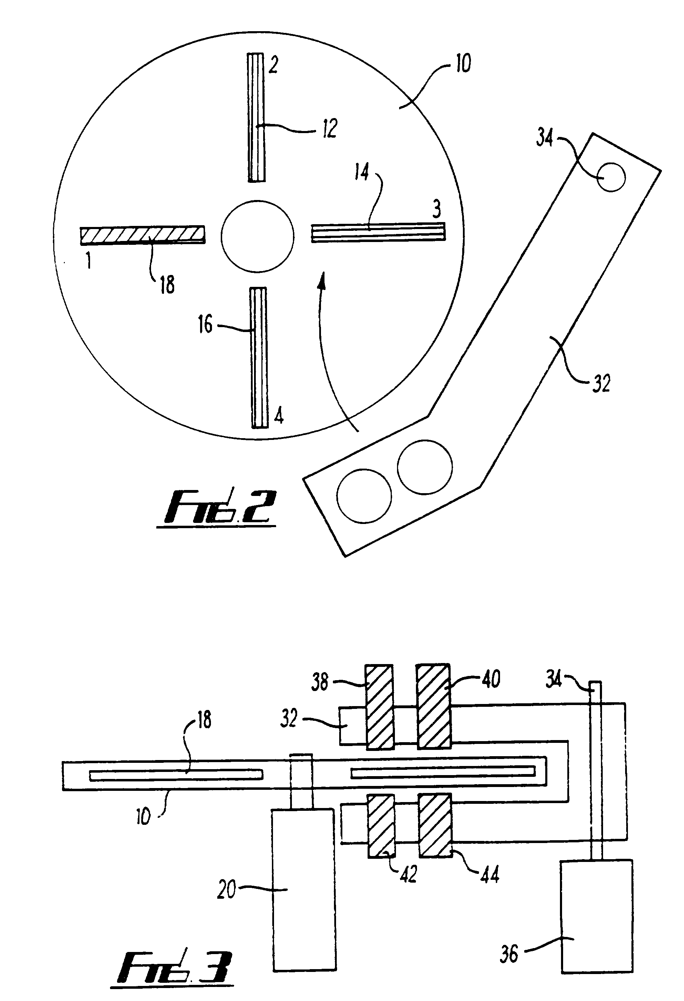

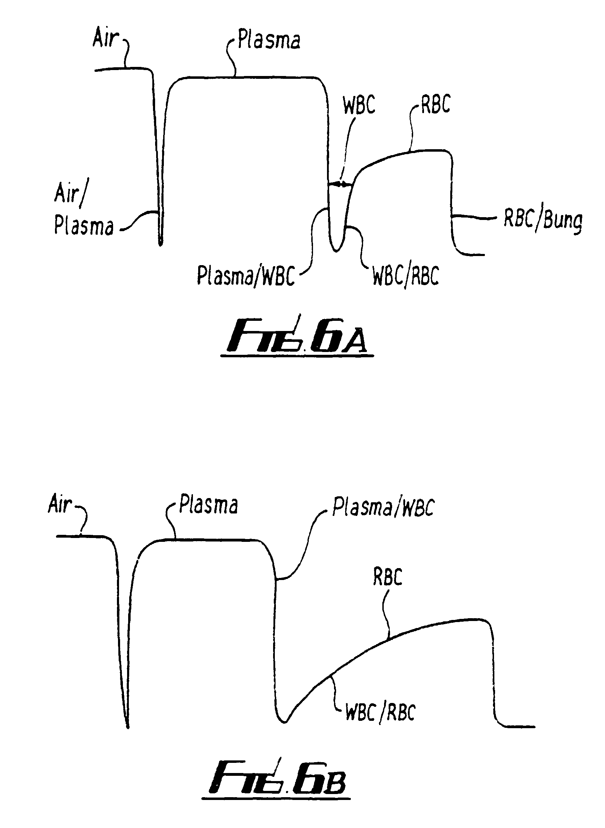

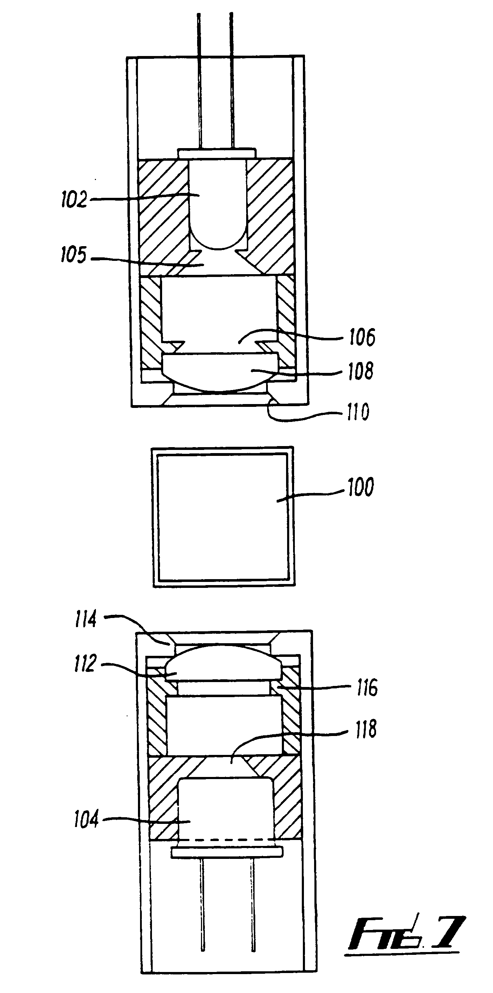

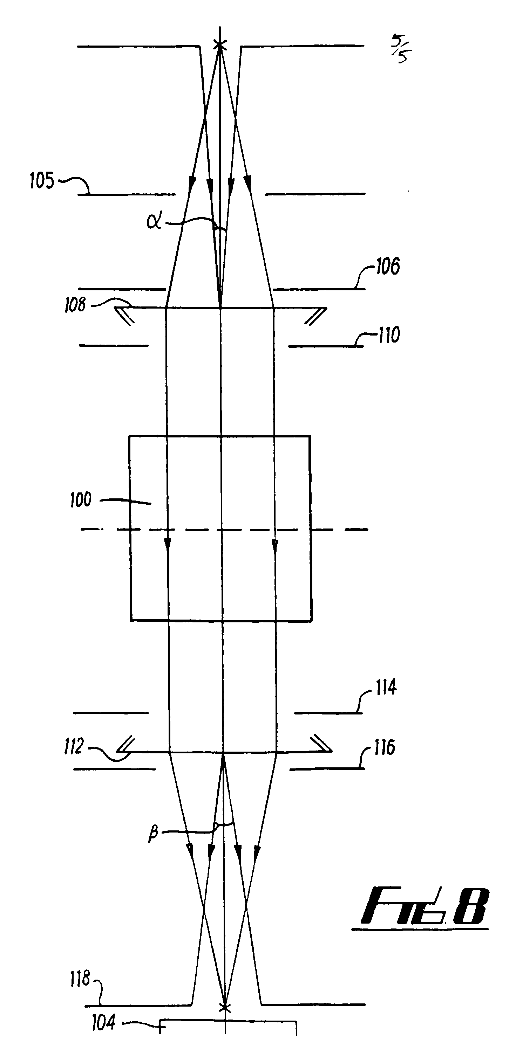

Referring to Figs. 2 and 3, a centrifuge rotor in the form of a disc 10 receives four haematocrit tubes 12, 14, 16 and 18 in radial alignment. The rotor is rotated by a drive motor 20 at a suitable speed to centrifuge the samples within the tubes 12-18. Typically, the samples are human or animal blood, and the centrifugation, as seen in Fig. 5, results in separation into packed red blood cells 22, packed white blood cells 24, and plasma 26. The outer end of the tube is closed with a bung 28, and the inner end contains a quantity of air at 30. Typically, centrifugation will take place at 11,000 rpm for 3 to 10 minutes.

Once the sample has been so separated, the rotor speed is reduced to about 1,000 rpm and a scanning arm 32 is moved across the disc 10 by rotation of its supporting shaft 34 by a motor and gear box 36. The scanning arm 32 is of generally U-shape, having an upper part mounting an infrared (IR) light source 38 and a visible light source 40, and a lower part mounting an IR detector 42 and a visible light detector 44.

As best seen in Fig. 4, each sample tube 12 etc is located in a slit 46 formed in the rotor 10, the slit 46 having a relatively wide top portion 46a and a relatively narrow under portion 46b joined by a shoulder on which the tube rests. The slit 46 thus allows light to pass from the source 38 or 42 to the associated detector 40 or 44.

The scanning process thus makes it possible to build up a curve defining the light absorption, at a given wavelength, along the length of each tube. One typical curve is shown in Fig. 6A.

In addition to the above percentage, a curve of this nature can provide further information. For example, in Fig. 6B the interface between white and red blood cell portions is poorly defined, which indicates the presence of nucleated red cells and reticulocytes.

A particular feature of the present invention is that scanning is carried out with both the IR source 38, suitably an IR laser, and with the visible light source described in more detail below.

The IR source 38 is used to detect the interfaces between the various fractions shown in Fig. 5. By detecting the relative positions of the interfaces, packed cell volume and white blood cell volume can be expressed as percentage of total volume. Blood cells and plasma are scattering media and are optically dense. We have established that a satisfactory resolution can be attained by using as a light source an IR laser with a wavelength of 785 mm and power of 1.5 mw.

Plasma may contain a number of components the presence and concentrations of which can provide crucial, early diagnostic information. Quantitative measurement of these components can enable the clinician quickly to plan further tests, and to avoid the adverse effects on certain tests arising from the presence of high concentrations of these components.

The plasma components normally of interest are bilirubin (icterus), triglycerides (lipaemia) and haemoglobin (haemolysis). In the past, these have generally been assessed by visual inspection, with unsatisfactory results. We have determined experimentally that the optimum wavelengths for detecting these three components are 625, 567 and 470 nm.

In the scanning arm of Figs. 2 and 3, the visible light source 42 is arranged to emit at these three frequencies sequentially. A suitable light source for doing so is a "Rainbow" LED by Ledtronics, ref. DIS-10024-002. This device emits light at three preselected frequencies, each in response to a respective signal.

In this manner, the plasma components of the samples are scanned for the three components of interest. It is not uncommon for all three to be present in the plasma of unwell patients. The present system allows all three to be measured at one time. The optical densities measured at each wavelength are converted to concentration units via calibration curves held in software.

The column of plasma is scanned along its length, rather than measured at a point. This makes it possible to measure component gradient, which may be of particular interest in lipaemia by providing information on the density of lipids present.

An important feature of this embodiment is the manner in which stability of the optical system is maintained. This is done by filling one haematocrit tube 12 with water and retaining it permanently on the rotor 10. This provides a datum value for 100% transmission, to which the sample signals are referenced. Sample optical densities are calculated using:

$$\text{Sample o.d.} = \log \frac{\text{reading of sample}}{\text{reading of water}}$$

The apparatus further includes a haemoglobin (Hb) photometer, which will now be described.

Referring to Fig. 7, a sample is placed in a transparent cuvette 100 and is exposed to monochromatic light from LED 102. The transmitted light is detected by a photocell 104 to derive a measure of optical density. It has been found that if a fixed quantity of whole blood is mixed with a fixed quantity of reagent, the optical density measurement is linearly related to Hb concentration over a range of interest. Preferably, a 20 ml sample is mixed with 3 ml of reagent and exposed to green light with a peak wavelength of about 560 nm. A suitable light source is a LED by Radiospares, catalogue no. 590-496, 563 nm peak, 250 mcd. This allows measurement of Hb concentration from 4 g/dl to a maximum of about 25 g/dl at which the optical density is about 0.8.

As seen in Fig. 7, light emitted by the LED 102 is passed through fixed stops 105 and 106 and collimated by lens 108. A further fixed stop 110 sets the area of the light beam applied to the cuvette 100. Light transmitted through the cuvette 100 is gathered by a collecting lens 112 mounted between equal aperture stops 114, 116 and passes to the photodetector 104 via a small diameter stop 118.

The geometry of this arrangement is shown schematically in Fig. 8. The use of an approximately parallel beam of light to illuminate the cuvette 100 means that small lateral and longitudinal misalignments of the cuvette have no significant detriment on accuracy. This arrangement also minimises the effects of non-uniformity of the sample itself or of the cuvette (for example, produced by fingerprints).

In order to minimise the effects of rotational misalignment, the stop 106 which forms the light source exit window is chosen to be less than the aperture of the detector lens 112 as determined by the stops 114, 116. The fact that the LED source 102 has a finite source size is unimportant provided that all the transmitted light arrives within the sensitive area of the detector 104; this is achieved by setting the geometry such that the angle β defined by the collecting lens 112 and the detector stop 118 is greater than the angle a defined by the collimating lens 108 and the emitting area of the LED 102.

The Hb photometer is maintained in stable operation by monitoring the LED output and compensating electronically for any drift. This may suitably be done by taking the value of optical density to be translated into Hb concentration as:

$$\text{optical density} = I/Io$$

where

I = detector output for the Hb cuvette, and
Io = the detector output with no cuvette present.

In a modification (not shown) provision is made for calibrating the Hb photometer immediately before or after each sample test. This may be done by taking a datum optical density reading on a cuvette containing a reference material (for example, water). It is contemplated that a reference cuvette could be held permanently in the instrument and could, for example, be spring biased into the optical path so as to be displaced by insertion of a sample cuvette. It might be necessary to use repeated calibration of this nature to minimise drift effects, such as the non-uniformity of LED output intensity caused by ambient temperature changes.

It is also contemplated to provide the photometer with a multiwavelength capability. For example, the 563 nm LED described with reference to Fig. 7 could be replaced by a Ledtronics "Rainbow" LED of the type described with reference to the centrifuge. This could be used to take measurements sequentially at 625 nm, 567 nm and 470 nm. Alternatively, it could be used, by controlling the currents to the red, blue and green emitting regions simultaneously, to produce a desired wavelength for a given test. Such polychromatic capability would be useful for a number of biochemical analyses other than blood.

The combination of the haemoglobin photometer and the photometric centrifuge in a single instrument with shared control and data handling gives a number of advantages. In particular,it allows ready derivation and recordal of diagnostic measures requiring data from both sources. A particular example of this is in the derivation of mean corpuscular haemoglobin concentration (MCHC), which is defined as:

$$\text{MCHC} = \frac{\text{Hb (g/dl)}}{\text{PCV (\%)}} \times 100 \text{ g/dl}$$

It will be seen that this can be readily derived from data supplied from the haemoglobin photometer and the IR detector.

It is however, equally within the scope of the invention as defined by the appended claims to use the photometric centrifuge as a stand-alone instrument.

**Claims**

1. An analysis apparatus comprising a centrifuge rotor (10) adapted to receive at least one sample in a transparent holder (12), the rotor being apertured to allow light to be transmitted through the sample and the rotor, and a scanning arm (32) arranged to traverse the rotor (10) and carrying light source means (38, 40) and light detector means (42, 44) on respective sides of the rotor (10);
characterised in that the light source means comprises a first light source (38) for detection of sample component interfaces and a second light source (40) adjacent to the first light source (38) for colourimetric inspection of at least one sample component.

2. Apparatus as claimed in Claim 1, wherein the first light source (38) is an infrared light source and the second light source (40) is a visible light source.

3. Apparatus as claimed in Claim 1 or Claim 2, wherein the infrared light source (38) is an infrared laser.

4. Apparatus as claimed in any one of the preceding Claims, wherein the sample is of blood, the visible light source (40) is used for examining blood plasma (26), and the visible light source (40) comprises three substantially monochromatic light sources of red, green and blue light respectively.

5. Apparatus as claimed in Claim 4, wherein the visible light source (40) comprises a single, multi-colour LED with the colours switched sequentially during the scanning process.

6. Apparatus as claimed in any one of the preceding Claims, further including a reference sample container on the rotor containing a reference material of known optical qualities, whereby signals representing light transmission through the sample are calibrated against signals representing light transmission through the reference material.

7. Apparatus as claimed in any preceding Claim, further comprising a haemoglobin photometer comprising means for receiving a transparent cell containing a blood sample mixed with a haemoglobin reagent, illumination means (102) for directing substantially monochromatic light at the sample cell (100), and detector means (104) arranged to receive light passing through the sample cell to generate a signal representing the optical density of the cell contents.

8. Apparatus as claimed in Claim 7, wherein the illumination means (102) comprises a LED operating at a peak wavelength of about 560 nm.

9. Apparatus as claimed in Claim 7 or Claim 8, wherein the illumination means (102) further comprises collimation means (108) causing the light directed at the sample cell to be substantially parallel.

10. Apparatus as claimed in any one of Claims 7 to 9, wherein the detector means (104) comprises a converging lens directing the transmitted light onto a photodiode.

11. Apparatus as claimed in any one of Claims 7 to 10, wherein the illumination means (102) and detector means (104) include optical stops (114, 116, 118) dimensioned and positioned such that light emitted from the whole emitting area of the LED is received within the active surface of the photodiode.

12. Apparatus as claimed in any one of Claims 7 to 11, further including calibration means operating to calibrate a detected optical density to a predetermined standard.

13. Apparatus as claimed in Claim 12, wherein the calibration means operates by comparing the output of the detector means with and without a sample cell in position.

14. A blood analyzer comprising a centrifuge (1) for separating a blood sample by centrifugation into packed red cells (22), packed white cells (24), and plasma (26); first optical means (38) for measuring the proportions of red cells, white cells and plasma in the sample; second optical means (40) arranged to measure the optical density of the plasma component at each of a plurality of visible wavelengths; photometer means (2) for measuring haemoglobin concentration in a sample of the same blood; and computer means (3) for deriving a number of parameters of interest from said measurements.

15. A method of analysing a blood sample using an analysis apparatus as claimed in Claim 2, the method including the steps of:

placing the sample in a haematocrit tube (12, 14, 16) on the centrifuge rotor (10);
placing a haematocrit tube (18) filled with water on the centrifuge rotor (10);
spinning the centrifuge rotor (10) at a first speed of 11,000 rpm to separate the fractions in the sample;
moving the scanning arm (32) across the rotor (10) while spinning the centrifuge rotor (10) at a second speed of 1000 rpm;

passing light from the first infrared light source (38) to a first detector (42) to detect the interfaces between the fractions in the sample along the length of the tube (12, 14, 16) by calibration of the signal received at the first detector through the sample with the signal received at the first detector through the water (18); passing light from the second visible light source (40) to a second detector (44) to measure the light absorption of the sample at a plurality of visible light wavelengths along the length of the tube (12, 14, 16) by calibration of the signal received at the second detector through the sample with the signal received at the second detector through the water (18).

**Patentansprüche**

1. Eine Analysevorrichtung, bestehend aus einem Zentrifugenrotor (10), der ausgeführt ist, um zumindest eine Probe in einem transparenten Behälter (12) aufzunehmen, wobei der Rotor mit Öffnungen versehen ist, damit Licht durch die Probe und den Rotor übertragen werden kann, und einem Abtastarm (32), der angeordnet ist, um den Rotor (10) und ein tragendes Lichtquellenmittel (38, 40) und ein Lichtdetektormittel (42, 44) auf den jeweiligen Seiten des Rotors (10) zu durchqueren; dadurch gekennzeichnet, daß das Lichtquellenmittel eine erste Lichtquelle (38) zur Detektion von Probenkomponentenzwischenflächen und eine zweite Lichtquelle (40), anliegend an die erste Lichtquelle (38), zur kolorimetrischen Inspektion von zumindest einer Probenkomponente umfaßt.

2. Vorrichtung gemäß Anspruch 1, wobei die erste Lichtquelle (38) eine Infrarotlichtquelle ist und die zweite Lichtquelle (40) eine sichtbare Lichtquelle ist.

3. Vorrichtung gemäß Anspruch 1 oder Anspruch 2, wobei die Infrarotlichtquelle (38) ein Infrarotlaser ist.

4. Vorrichtung gemäß einem der vorhergehenden Ansprüche, wobei die Probe aus Blut besteht, wobei die sichtbare Lichtquelle (40) verwendet wird, um Blutplasma (26) zu untersuchen, und die sichtbare Lichtquelle (40) drei im wesentlichen monochromatische Lichtquellen aus rotem, grünem bzw. blauem Licht umfaßt.

5. Vorrichtung gemäß Anspruch 4, wobei die sichtbare Lichtquelle (40) eine einzige, mehrfarbige Leuchtdiode ist, wobei die Farben während des Abtastverfahrens aufeinanderfolgend gewechselt werden.

6. Vorrichtung gemäß einem der vorhergehenden Ansprüche, die weiters einen Referenzprobenbehälter auf dem Rotor umfaßt, der ein Referenzmaterial mit bekannten optischen Qualitäten enthält, wodurch Signale, welche die Lichtübertragung durch die Probe darstellen, mit Signalen, welche die Lichtübertragung durch das Referenzmaterial darstellen, kalibriert werden.

7. Vorrichtung gemäß einem der vorhergehenden Ansprüche, weiters bestehend aus einem Hämoglobinphotometer, der ein Mittel zum Aufnehmen einer transparenten Zelle, die eine Blutprobe enthält, welche mit einem Hämoglobinreagens gemischt ist, einem Beleuchtungsmittel (102) zum Richten von im wesentlichen monochromatischem Licht auf die Probezelle (100) und einem Detektormittel (104), das so angeordnet ist, um durch die Probezelle hindurchgehendes Licht aufzunehmen, damit ein Signal erzeugt wird, das die optische Dichte der Zelleninhalte darstellt, umfaßt.

8. Vorrichtung gemäß Anspruch 7, wobei das Beleuchtungsmittel (102) eine Leuchtdiode umfaßt, die auf einer Höchstwellenlänge von ungefähr 560 nm läuft.

9. Vorrichtung gemäß Anspruch 7 oder Anspruch 8, wobei das Beleuchtungsmittel (102) weiters ein Kollimationsmittel (108) umfaßt, das bewirkt, daß das auf die Probezelle gerichtete Licht im wesentlichen parallel verläuft.

10. Vorrichtung gemäß einem der Ansprüche 7 bis 9, wobei das Detektormittel (104) eine Sammellinse umfaßt, die das übertragene Licht auf eine Photodiode richtet.

11. Vorrichtung gemäß einem der Ansprüche 7 bis 10, wobei das Beleuchtungsmittel (102) und das Detektormittel (104) optische Blenden (114, 116, 118) umfaßt, die so dimensioniert und angeordnet sind, daß das von der gesamten emittierenden Fläche der Leuchtdiode emittierte Licht innerhalb der aktiven Oberfläche der Photodiode aufgenommen wird.

12. Vorrichtung gemäß einem der Ansprüche 7 bis 11, weiters bestehend aus einem Kalibriermittel, das so funktioniert, daß es eine ermittelte optische Dichte auf einen vorbestimmten Standard kalibriert.

13. Vorrichtung gemäß Anspruch 12, wobei das Kalibriermittel so funktioniert, indem es die abgegebene Leistung des Detektormittels mit einer positionierten Probezelle und ohne eine solche vergleicht.

14. Ein Blutanalysiergerät, bestehend aus einer Zentrifuge (1) zur Teilung einer Blutprobe durch Zentrifugieren in gepackte rote Zellen (22), gepackte weiße Zellen (24) und Plasma (26); einem ersten optischen Mittel (38) zur Messung der Verhältnisse von roten Zellen, weißen Zellen und Plasma in der Probe; einem zweiten optischen Mittel (40), das so angeordnet ist, daß es die optische Dichte der Plasmakomponente bei jeder der Vielzahl von sichtbaren Wellenlängen misst; einem Photometermittel (2) zur Messung der Hämoglobinkonzentration in einer Probe desselben Blutes; und einem Rechnermittel (3) zur Ableitung einer Anzahl wichtiger Parameter von diesen Messungen.

15. Ein Verfahren zur Analyse einer Blutprobe unter Verwendung eines Analysiergerätes gemäß Anspruch 2, wobei das Verfahren folgende Schritte umfaßt:

   Eingeben der Probe in ein Hämatokritröhrchen (12, 14, 16) auf dem Zentrifugenrotor (10);

   Stellen eines mit Wasser gefüllten Hämatokritröhrchens (18) auf den Zentrifugenrotor (10);

   Schleudern des Zentrifugenrotors (10) bei einer ersten Geschwindigkeit von 11000 U/min, um die Fraktionen in der Probe zu teilen;

   Bewegen des Abtastarms (32) über den Rotor (10), während der Zentrifugenrotor (10) bei einer zweiten Geschwindigkeit von 1000 U/min geschleudert wird;

   Leiten von Licht von der ersten Infrarotlichtquelle (38) zu einem ersten Detektor (42), um die Schnittflächen zwischen den Fraktionen in der Probe entlang der Länge des Röhrchens (12, 14, 16) durch Kalibrieren des Signals, das beim ersten Detektor durch die Probe erhalten wurde, mit dem Signal, das am ersten Detektor durch das Wasser (18) erhalten wurde, zu ermitteln;

   Leiten von Licht von der zweiten sichtbaren Lichtquelle (40) zu einem zweiten Detektor (44), um die Lichtabsorption der Probe bei einer Vielzahl von sichtbaren Lichtwellenlängen entlang der Länge des Röhrchens (12, 14, 16) durch Kalibrieren des Signals, das am zweiten Detektor durch die Probe erhalten wurde, mit dem Signal, das am zweiten Detektor durch das Wasser (18) erhalten wurde, zu messen.

**Revendications**

1. Un appareil d'analyse comprenant un rotor de centrifugeuse (10) adapté pour recevoir au moins un échantillon dans un récipient transparent (12), des ouvertures étant ménagées dans le rotor pour permettre à de la lumière d'être transmise au travers de l'échantillon et du rotor, et un bras de balayage (32) agencé pour traverser le rotor (10) et portant un moyen de sources de lumière (38, 40) et un moyen détecteur de lumière (42, 44) sur des côtés respectifs du rotor (10) ;
caractérisé en ce que le moyen de sources de lumière comprend une première source de lumière (38) destinée à la détection d'interfaces de composants de l'échantillon et une seconde source de lumière (40) adjacente à la première source de lumière (38) destinée à l'inspection colorimétrique d'au moins un composant de l'échantillon.

2. Appareil selon la revendication 1, dans lequel la première source de lumière (38) est une source de lumière infrarouge et la seconde source de lumière (40) est une source de lumière visible.

3. Appareil selon la revendication 1 ou la revendication 2, dans lequel la source de lumière infrarouge (38) est un laser infrarouge.

4. Appareil selon une quelconque des revendications précédentes, dans lequel le prélèvement est un échantillon de sang, la source de lumière visible (40) est utilisée pour examiner du plasma sanguin (26) et la source de lumière visible (40) comprend trois sources de lumière sensiblement monochromatique consistant en lumière rouge, verte et bleue respectivement.

5. Appareil selon la revendication 4, dans lequel la source de lumière visible (40) comprend une seule DEL multichrome dont les couleurs sont commutées de façon séquentielle durant le processus de balayage.

6. Appareil selon une quelconque des revendications précédentes, comportant de plus un conteneur d'échantillon témoin sur le rotor contenant une matière témoin de qualités optiques connues, grâce à quoi des signaux représentant la transmission de lumière à travers l'échantillon sont étalonnés sur des signaux représentant la transmission de lumière au travers de la matière témoin.

7. Appareil selon n'importe quelle revendication précédente, comprenant de plus un photomètre d'hémoglobine comprenant un moyen destiné à recevoir une cellule transparente contenant un échantillon de sang mélangé à un réactif d'hémoglobine, un moyen d'illumination (102) destiné à diriger de la lumière sensiblement monochromatique sur la cellule d'échantillon (100) et un moyen détecteur (104) agencé pour recevoir de la lumière passant au travers de la cellule d'échantillon pour générer un signal représentant la densité optique du contenu de la cellule.

8. Appareil selon la revendication 7, dans lequel le moyen d'illumination (102) comprend une DEL fonctionnant à une longueur d'onde de crête d'environ 560 nm.

9. Appareil selon la revendication 7 ou la revendication 8, dans lequel le moyen d'illumination (102) comprend de plus un moyen de collimation (108) amenant la lumière dirigée sur la cellule d'échantillon à être sensiblement parallèle.

10. Appareil selon une quelconque des revendications 7 à 9, dans lequel le moyen détecteur (104) comprend une lentille convergente dirigeant la lumière transmise sur une photodiode.

11. Appareil selon une quelconque des revendications 7 à 10, dans lequel le moyen d'illumination (102) et le moyen détecteur (104) comportent des butées optiques (114, 116, 118) dimensionnées et positionnées de telle sorte que la lumière émise depuis la zone d'émission tout entière de la DEL est reçue au sein de la surface active de la photodiode.

12. Appareil selon une quelconque des revendications 7 à 11, comportant de plus un moyen d'étalonnage fonctionnant pour étalonner une densité optique détectée à une norme prédéterminée.

13. Appareil selon la revendication 12, dans lequel le moyen d'étalonnage fonctionne en comparant la sortie du moyen détecteur avec et sans cellule d'échantillon en position.

14. Un analyseur de sang comprenant une centrifugeuse (1) destinée à séparer un échantillon de sang par centrifugation en amas de cellules rouges (22), en amas de cellules blanches (24) et en plasma (26) ; un premier moyen optique (38) destiné à mesurer les proportions de cellules rouges, de cellules blanches et de plasma dans l'échantillon ; un second moyen optique (40) agencé pour mesurer la densité optique du composant de plasma à chaque longueur d'onde visible parmi une pluralité de celles-ci ; un moyen de photomètre (2) destiné à mesurer la concentration en hémoglobine dans un échantillon du même sang ; et un moyen informatique (3) destiné à dériver un certain nombre de paramètres d'intérêt desdites mesures.

15. Un procédé pour analyser un échantillon de sang à l'aide d'un appareil d'analyse selon la revendication 2, le procédé comportant les étapes consistant :

à placer l'échantillon dans un tube d'hématocrite (12, 14, 16) sur le rotor de centrifugeuse (10) ;

à placer un tube d'hématocrite (18) rempli d'eau sur le rotor de centrifugeuse (10) ;

à faire tourner le rotor de centrifugeuse (10) à une première vitesse de 11 000 tr/min pour séparer les fractions dans l'échantillon ;

à faire déplacer le bras de balayage (32) sur le rotor (10) tout en faisant tourner le rotor de centrifugeuse (10) à une seconde vitesse de 1 000 tr/min ;

à faire passer de la lumière de la première source de lumière infrarouge (38) à un premier détecteur (42) pour détecter les interfaces entre les fractions de l'échantillon sur la longueur du tube (12, 14, 16) par étalonnage du signal reçu au premier détecteur au travers de l'échantillon avec le signal reçu au premier détecteur au travers de l'eau (18);

à faire passer de la lumière de la seconde source de lumière visible (40) à un second détecteur (44) pour mesurer l'absorption de lumière de l'échantillon à une pluralité de longueurs d'onde de lumière visible sur la longueur du tube (12, 14, 16) par étalonnage du signal reçu au second détecteur au travers de l'échantillon avec le signal reçu au second détecteur au travers de l'eau (18).

-2-

-1-

IR    VIS

-3-

-5-

-6-

-4-

_Fig.1_

40

12    46a    10

46b

44

_Fig. 4_

28    22    24    26    30

_Fig. 5_

EP 0 795 129 B1

Fig.2

Fig.3

10

Air
Plasma
WBC
RBC
Air/
Plasma
Plasma/WBC
WBC/RBC
RBC/Bung

FIG. 6A

Air
Plasma
Plasma/WBC
RBC
WBC/RBC

FIG. 6B

FIG 7

FIG. 8